# EUROPEAN PATENT APPLICATION

(11) **EP 4 721 707 A2**
(43) Date of publication of application: **08.04.2026**
(21) Application number: 26158562.4
(22) Date of filing: 21.10.2020
(51) Int. Cl.: A61F 2/40

(54) **ORTHOPEDIC IMPLANT SYSTEM WITH AUGMENTATION DEVICE AND METHODS OF USE**

(30) Priority: 24.10.2019 US 201962925440 P
(62) Divisional of application: 20878453.8
(71) Applicant: DePuy Ireland Unlimited Company, Ringaskiddy, County Cork (IE)
(72) Inventor: HODOREK, Brian C., Winona Lake, 46590 (US); PURDY, Matt J., Winona Lake, 46590 (US); PARROTT, Russ M., Winona Lake, 46590 (US); WIATER, J. Michael, Beverly Hills, 48025 (US); MURTHI, Anand M., Baltimore, 21218 (US); SMITH, Matthew J., Columbia, 65203 (US); CUFF, Derek J., Venice, 34292 (US); JAWA, Andrew, Cambridge, 02139 (US); AUSTIN, Luke, Haddonfield, 08033 (US)
(74) Representative: Murgitroyd & Company

(57) **Abstract**

An implant system includes a base plate having at least one thru-hole. A central screw is operable to extend from a lower surface of the base plate and anchor the base plate to a bone. At least one augmentation device is operable to extend through the at least one thru-hole. The augmentation device includes a post having a first end, a second end and a length sized to enable the augmentation device to traverse a defect in the bone when the base plate is anchored to the bone. A fastener portion is positioned at the first end of the post. The fastener portion is operable to fasten to the base plate. The footing position may be positioned at the second end of the post. The footing portion is operable to abut against a surface of the bone within the defect when the fastener portion is fastened to the base plate.

## Description

### CROSS REFERENCE TO RELATED APPLICATION

This application perfects and claims priority benefit to U.S. Provisional Patent Application No. 62/925,440, filed October 24, 2019, entitled "Orthopedic Implant System With Augmentation Device and Methods Of Use," which application is hereby incorporated herein by reference in its entirety.

### FIELD OF THE INVENTION

The present invention relates generally to orthopedic surgery, implant systems used for replacing an articulation surface in a joint, such as, for example, shoulder prostheses. More specifically, but not exclusively, the present invention relates to glenoid implant systems for shoulder arthroplasties having augmentation devices to provide stability to a glenoid base plate when implanted over a glenoid deficiency, as well as methods for using the same.

### BACKGROUND OF THE INVENTION

Oftentimes during orthopedic surgery, a surgeon will encounter defects in a bone that the surgeon is operating on. For example, a surgeon will often encounter bone defects, such as voids or glenoid deficiencies, in various areas of a glenoid cavity during anatomic or reverse arthroplasty. Even after reaming preparation, defects may exist under the backsides of base plates of an orthopedic glenoid implant. Improper support may result in loosening and failure of the implant.

If a surgeon wanted complete backside support of the base plate, he could continue reaming deeper until one-hundred percent backside coverage is achieved (known as eccentric reaming). However, this often results in excessive removal of cortical bone (or cortical layer), i.e., the dense outer surface bone that forms a protective layer around the internal cavity of the bone. Studies have shown that cortical bone removal correlates with loosening of the implant over time.

Various augmented glenoid base plates exist that have predetermined shapes on their backsides. The predetermined shapes are designed to more closely fit various types of glenoid defects. However, these augmented base plates require extensive preparation of the glenoid cavity to create the angles and depths required to receive the augmented base plates. In addition, a large inventory of instruments and augmented implants with various backside shapes need to be brought into the operating room to cover whatever glenoid deficiency the surgeon may encounter. Moreover, the surgeon cannot determine which augmented base plate to use until the patient's glenoid is exposed.

Patient specific base plates, having a backside geometry that conforms to the patient's glenoid anatomy often require less preparation than preformed augmented base plates. However, to make such patient specific base plates, computer tomography (CT) scans are required on the patient to determine the shape of the customized base plate. This significantly increases the time and cost to the surgical procedure when compared to a conventional shoulder arthroplasty surgery. Additionally, custom devices are expensive and require an increased investment of time and resources on behalf of the company supplying the device.

Accordingly, there is a need for a base plate design and procedure that can provide modular stability to a base plate when bone defects are present without excessive bone removal. Additionally, there is a need for a base plate design and procedure that can provide such stability over a variety of defects with the same standard base plate. Moreover, there is a need to provide such stability without the need to manufacture a custom base plate that matches the patient's bone defects.

### SUMMARY OF THE INVENTION

Aspects of the present invention provides implant systems with augmentation devices for replacing an articulation surface in a joint, such as, for example, shoulder prostheses. The augmentation device is used to provide stability to a base plate of an implant system when the base plate is located proximate to a defect, such as a void, in a bone. The present invention also provides for methods for using the implant systems.

In an aspect, provided herein is an implant system including a base plate having at least one thru-hole. A central screw is operable to extend from a lower surface of the base plate and anchor the base plate to a bone. At least one augmentation device is operable to extend through the at least one thru-hole. The augmentation device includes a post having a first end, a second end and a length sized to enable the augmentation device to traverse a defect in the bone when the base plate is anchored to the bone. A fastener portion is positioned on the first end of the post. The fastener portion is operable to fasten to the base plate. A footing portion is positioned on the second end of the post. The footing portion is operable to abut against a surface of the bone within the defect when the fastener portion is fastened to the base plate.

In another aspect, provided herein is an implant system including a base plate having a plurality of threaded peripheral holes disposed circumferentially around a central region of the base plate. A central screw is operable to extend from a lower surface of the base plate and threadingly anchor the base plate to a bone. At least one augmentation device is operable to extend through a first peripheral hole of the plurality of peripheral holes. The augmentation device includes a post having a first end, a second end and a length sized to enable the augmentation device to traverse a defect in the bone when the base plate is anchored to the bone. A threaded fastener portion of the augmentation device is positioned on the first end of the post. The fastener portion has threads configured to engage with threads of the first peripheral hole. A footing portion of the augmentation device is positioned on the second end of the post. The footing portion is operable to abut against a surface of the bone within the defect when the fastener portion is fastened to the base plate.

In another aspect, provided herein is an augmentation device of an implant system, wherein the implant system has a base plate that is operable to be anchored to a bone. The augmentation device includes a post that is operable to extend through a thru-hole of the base plate. The post has a first end, a second end and a length sized to enable the augmentation device to traverse a defect in the bone when the base plate is anchored to the bone. A fastener portion is positioned on the first end of the post. The fastener portion is operable to fasten to the base plate. A footing portion is positioned on the second end of the post. The footing portion is operable to abut against a surface of the bone within the defect when the fastener portion is fastened to the base plate.

In another aspect provided herein is a method of surgically implanting an orthopedic implant into a bone. The method includes surgically exposing the surface of the bone. A circular surface is reamed into a portion of the bone. A center bore hole is drilled into the portion of the bone. The center bore hole and circular surface are concentric. A thru-hole of a base plate of an implant system is oriented over a defect in the bone. The base plate is inserted into the center bore hole such that the base plate is press-fit into the center bore hole and the thru-hole is located over the defect. An augmentation device is extended through the thru-hole. The augmentation device is fastened to the base plate such that a foot portion of the augmentation device abuts against, and does not penetrate, the surface of the bone within the defect.

These, and other objects, features and advantages of this invention will become apparent from the following detailed description of the various aspects of the invention taken in conjunction with the accompanying drawings.

### BRIEF DESCRIPTION OF THE DRAWINGS

The accompanying drawings, which are incorporated in and constitute a part of the specification, illustrate embodiments of the invention and together with the detailed description herein, serve to explain the principles of the invention. The drawings are only for purposes of illustrating preferred embodiments and are not to be construed as limiting the invention. It is emphasized that, in accordance with the standard practice in the industry, various features are not drawn to scale. In fact, the dimensions of the various features may be arbitrarily increased or reduced for clarity of discussion. The foregoing and other objects, features and advantages of the invention are apparent from the following detailed description taken in conjunction with the accompanying drawings in which:
FIG. 1 depicts a side perspective view of a defective glenoid cavity of a scapula bone, in accordance with an aspect of the present invention;
FIG. 2 depicts a side perspective view of an orthopedic implant system, in accordance with an aspect of the present invention;
FIG. 3 depicts an exploded side view of the orthopedic implant system of FIG. 2, in accordance with an aspect of the present invention;
FIG. 4 depicts a top perspective view of a base plate of the orthopedic implant system of FIG. 2, in accordance with an aspect of the present invention;
FIG. 5 depicts a bottom perspective view of the base plate of FIG. 2, in accordance with an aspect of the present invention;
FIG. 6 depicts a side perspective view of a central screw of the orthopedic implant system of FIG. 2, in accordance with an aspect of the present invention;
FIG. 7 depicts a side perspective view of a coupling member of the orthopedic implant system of FIG. 2, in accordance with an aspect of the present invention;
FIG. 8 depicts a top perspective view of an embodiment of an augmentation device of the orthopedic implant system of FIG. 2 having a flat bottom footing portion, in accordance with an aspect of the present invention;
FIG. 9 depicts a side view of the augmentation device of FIG. 8, in accordance with an aspect of the present invention;
FIG. 10 depicts a side perspective view of another embodiment of an augmentation device having an arcuate bottom footing portion, in accordance with an aspect of the present invention;
FIG. 11 depicts a side perspective view of another embodiment of an augmentation device having a conical bottom footing portion, in accordance with an aspect of the present invention;
FIG. 12 depicts a side perspective view of another embodiment of an augmentation device having a threaded bottom footing portion, in accordance with an aspect of the present invention;
FIG. 13 depicts block diagram of a method of surgically preparing a bone to receive an orthopedic implant system, in accordance with an aspect of the present invention;
FIG. 14 depicts a bottom perspective view of a bone reamer used to prepare a top surface of a glenoid cavity to accept an orthopedic implant system, in accordance with the present invention;
FIG. 15 depicts a top perspective view of a defective glenoid cavity, wherein the top surface of the glenoid cavity has been prepared to accept the orthopedic implant system of FIG. 2, in accordance with an aspect of the present invention;
FIG. 16 depicts a side perspective view of the defective glenoid cavity of FIG. 15, in accordance with an aspect of the present invention;
FIG. 17 depicts a side perspective view of a base plate of an orthopedic implant system being inserted into the prepared glenoid cavity of FIG. 15 with an insertion tool, in accordance with an aspect of the present invention;
FIG. 18 depicts a side perspective view of the orthopedic implant system of FIG. 2 implanted into the glenoid cavity of FIG. 17, in accordance with an aspect of the present invention;
FIG. 19 depicts a top perspective view of the orthopedic implant system of FIG. 2 having a glenosphere disposed over the base plate for use in a reverse shoulder arthroplasty, in accordance with an aspect of the present invention;
FIG. 20 depicts a bottom perspective view of the orthopedic implant system of FIG. 19, in accordance with an aspect of the present invention;
FIG. 21 depicts a side perspective view of the orthopedic implant system of FIG. 2, having a glenoid liner disposed over the base plate for use in an anatomic shoulder arthroplasty, in accordance with an aspect of the present invention;
FIG. 22 depicts a side perspective view on another example of an orthopedic implant system, in accordance with aspects of the present invention; and
FIG. 23 depicts a side exploded view of the orthopedic implant system of FIG. 22, in accordance with aspects of the present invention.

### DETAILED DESCRIPTION FOR CARRYING OUT THE INVENTION

Generally stated, disclosed herein are implant systems and methods of making the same. Further, surgical methods for using the implant systems are discussed.

In this detailed description and the following claims, the words proximal, distal, anterior, posterior, medial, lateral, superior and inferior are defined by their standard usage for indicating a particular part of a bone or implant according to the relative disposition of the natural bone or directional terms of reference. For example, "proximal" means the portion of a device or implant nearest the torso, while "distal" indicates the portion of the device or implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure.

As used herein, the word "exemplary" or "illustrative" means "serving as an example, instance, or illustration." Any implementation described herein as "exemplary" or "illustrative" is not necessarily to be construed as preferred or advantageous over other implementations. Moreover, in the present description, the terms "upper", "lower", "left", "rear", "right", "front", "vertical", "horizontal", and derivatives thereof shall relate to the invention as oriented in the first figure of each embodiment.

Similarly, positions or directions may be used herein with reference to anatomical structures or surfaces. For example, as the current implant systems (or implants), devices, systems and methods are described herein with reference to use with the bones of the shoulder, the bones of the shoulder and upper arm may be used to describe the surfaces, positions, directions or orientations of the implant systems, devices, systems and methods. Further, the implant systems, devices, systems and methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the implant systems, devices, systems and methods, and the aspects, components, features and the like thereof, described and/or illustrated herein may be changed, varied, modified, reconfigured or otherwise altered for use or association with another side of the body for a same or similar purpose without departing from the spirit and scope of the invention. For example, the implant systems, devices, systems and methods, and the aspects, components, features and the like thereof, described herein with respect to the right shoulder may be mirrored so that they likewise function with the left shoulder and vice versa. Further, the implant systems, devices, systems and methods, and the aspects, components, features and the like thereof, disclosed herein are described with respect to the shoulder for brevity purposes, but it should be understood that the implant systems, devices, systems and methods may be used with other bones of the body having similar structures, for example the lower extremity, and more specifically, with the bones of the ankle, foot, and leg.

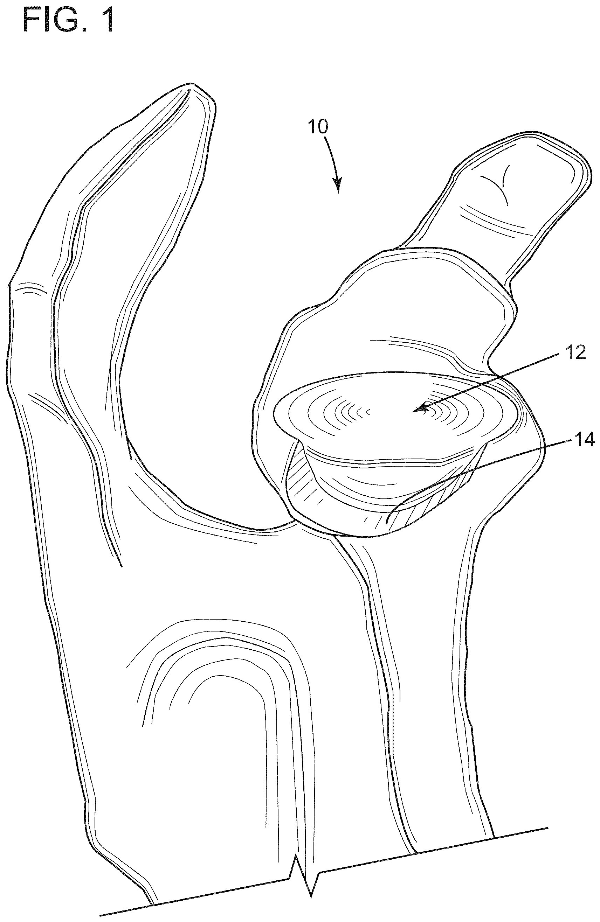

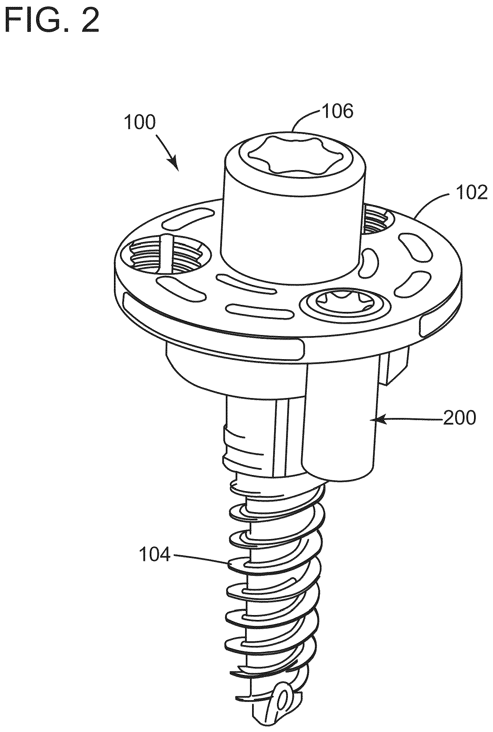

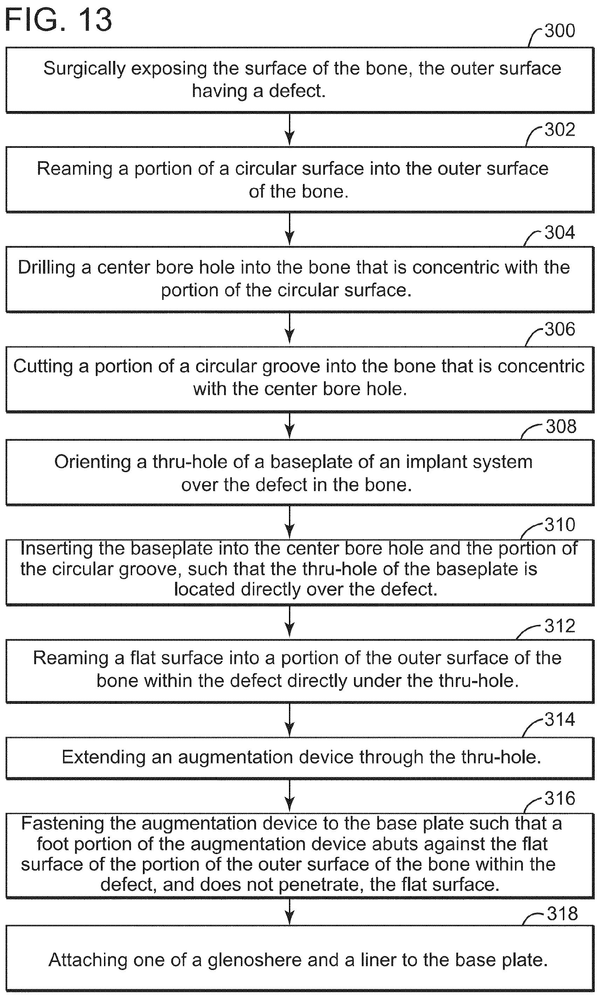

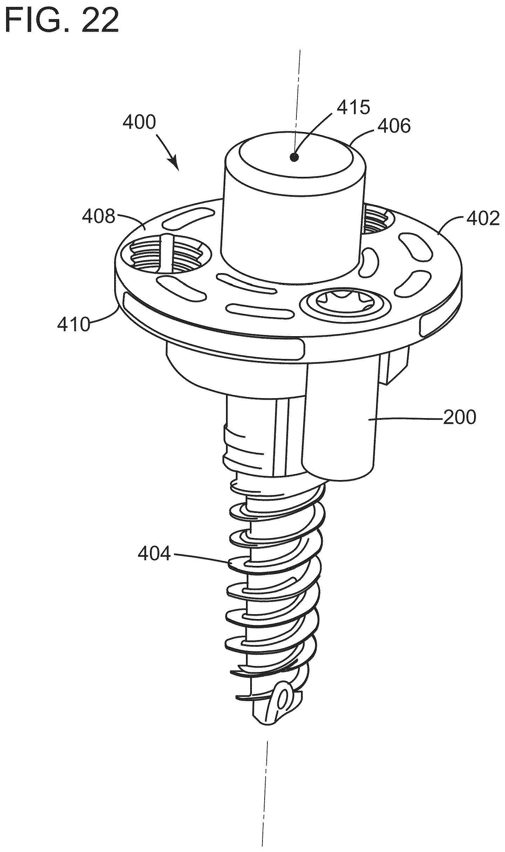

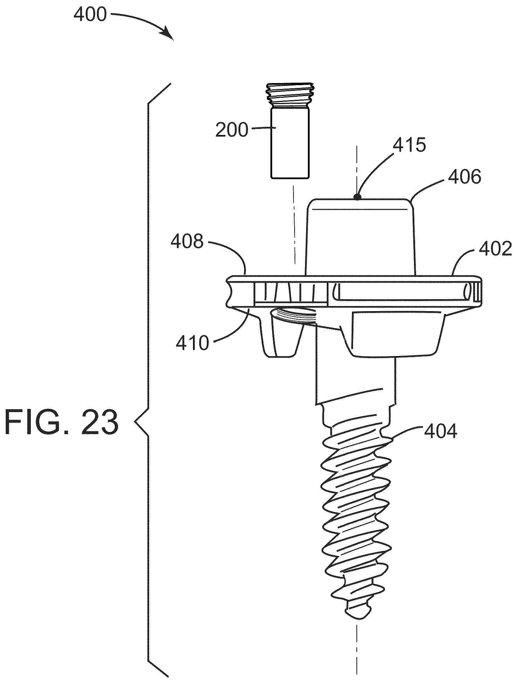

Referring to the drawings, like reference numerals are used to indicate like or analogous components throughout the several views. Particularly, FIG. 1 illustrates one example of a defective glenoid cavity. FIGS. 2-12 illustrate various views of an implant system in accordance with aspects of the present invention. Additionally, FIGS. 13-18 illustrate various steps and devices utilized in a method of preparing a bone for an orthopedic implant system in accordance with aspects of the present invention. FIGS. 22 and 23 illustrate various views of an example of another embodiment of an implant system, wherein a base plate and central screw of the implant system are formed as a single monoblock construct.

Referring to FIG. 1, an example of a lateral perspective view of a defective glenoid cavity 12 of a scapula 10 is depicted in accordance with aspects of the present invention. The glenoid cavity 12 includes at least one bone defect 14 that has been developed over time in a patient. For example, the bone defect 14 may be, without limitation, a void. The defect consists of a depression beyond the normal anatomy of the glenoid cavity.

Though the example of a defective scapula 10 illustrated in FIG. 1 shows one defect 14, two or more defects are also possible. Such defects may vary in depth and size depending on the conditions that caused them. Additionally, though the figures herein illustrate a glenoid cavity 12 with a defect, other bones in the human anatomy may also have similar defects that may be treatable within the scope of the present invention.

Such defects provide irregular surfaces in the glenoid cavity 12, thereby reducing structural support for the backside of base plates of orthopedic implant systems (or implants), even after reaming the glenoid cavity's articulating surface. If a surgeon wanted complete backside support of the base plate, the surgeon would have to continue reaming deeper until one-hundred percent backside coverage is achieved (known as eccentric reaming). However, this often results in excessive removal of the cortical layer. Studies have shown that cortical layer removal correlates with loosening of the implant over time. The cortical layer (or cortical bone) herein is the dense outer surface of the bone that forms a protective layer around the internal cavity of the bone.

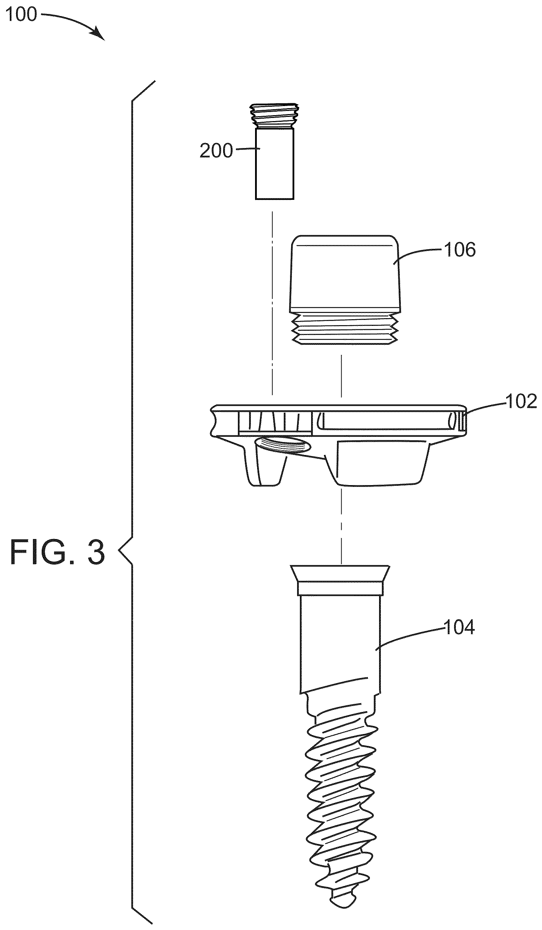

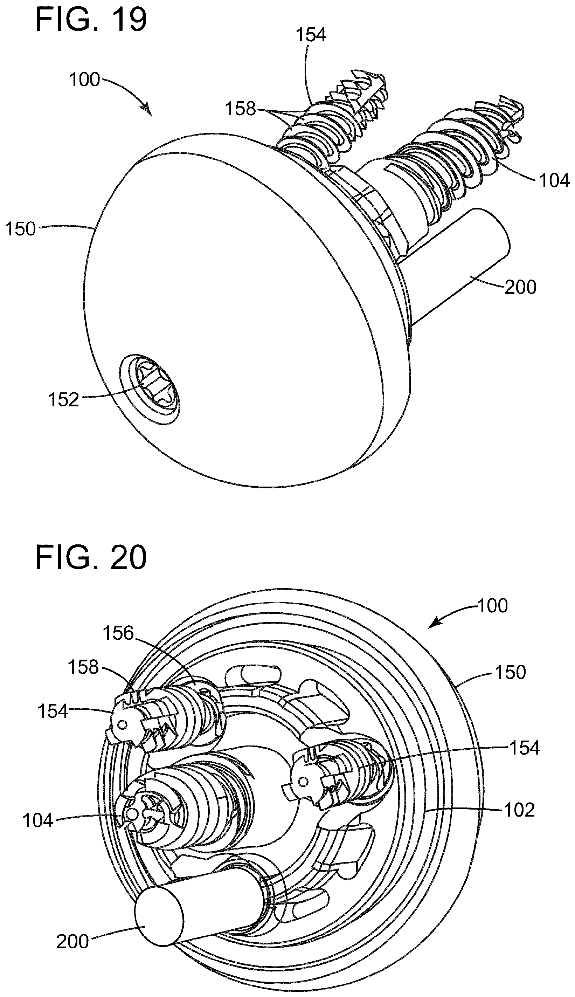

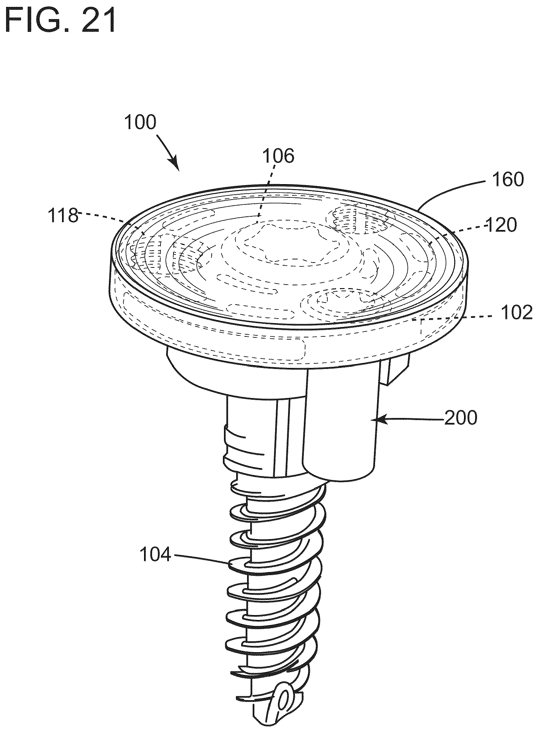

Referring to FIGS. 2 and 3, a side perspective view (FIG. 2) and an exploded view (FIG. 3) of an orthopedic implant system 100 is depicted, in accordance with an aspect of the present invention. Implant system 100 includes a base plate 102, a central screw 104, a coupling member 106, and at least one augmentation device 200. The implant system 100 may also include one or more peripheral screws 154 (seen in FIGS. 19 and 20). Further, the implant system may include a glenoid sphere 150 (seen in FIGS. 19 and 20) for use in a reverse shoulder arthroplasty and/or a glenoid liner 160 (seen in FIG. 21) for use in an anatomic shoulder arthroplasty.

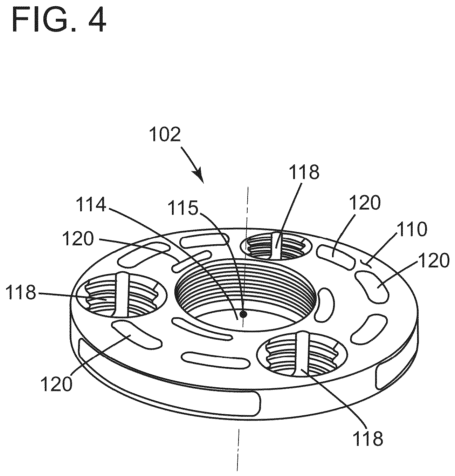

In the example of the implant system 100 illustrated in FIGS. 2 and 3, the base plate 102, central screw 104 and coupling member 106 are separate pieces. Additionally, the central screw 104 is separable from the base plate 102 and operable to extend through a central bore 114 (see FIG. 4), which is positioned in a central region 115 of the base plate 102. However, another example of an implant system 400 is illustrated in FIGS. 22 and 23 that is also within the scope of the present invention. In implant system 400, the base plate 402, central screw 404 and coupling member 406, may be integrally connected as a single monobloc construct.

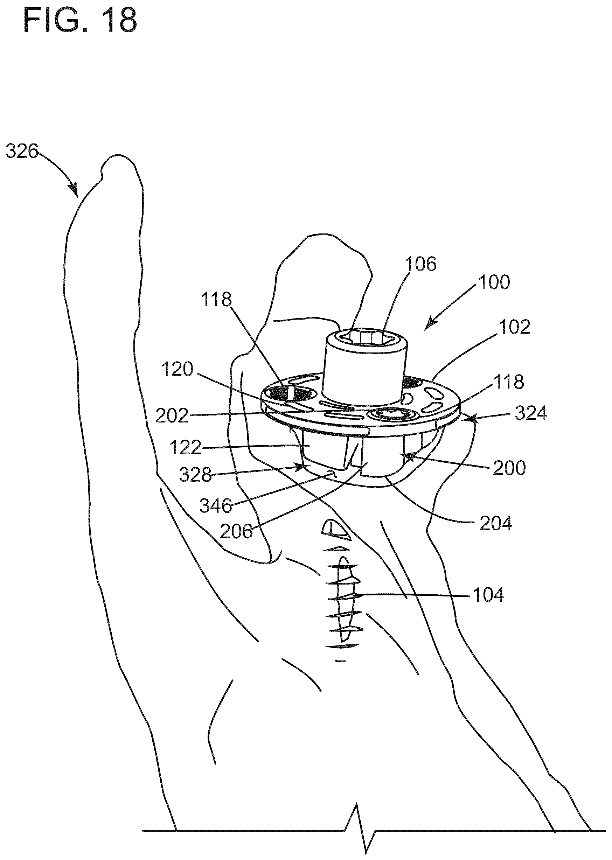

As will be explained in greater detail herein, the augmentation device 200 provides stability to the implant system 100, when the implant system is inserted into a glenoid cavity 324 having a bone defect 328, such as a void (seen in FIG. 18). The augmentation device 200 can be fastened to the base plate 102 and extend into the bone defect 14 such that a footing end of the augmentation device abuts against the surface of the bone within the defect 14. The bone within the defect 14 may include a hard bone, for example, cortical bone. The augmentation 200 can provide support to the base plate 102, much like the legs of a table provide support for the table, with minimal bone removal from the surface of the bone within the defect 14.

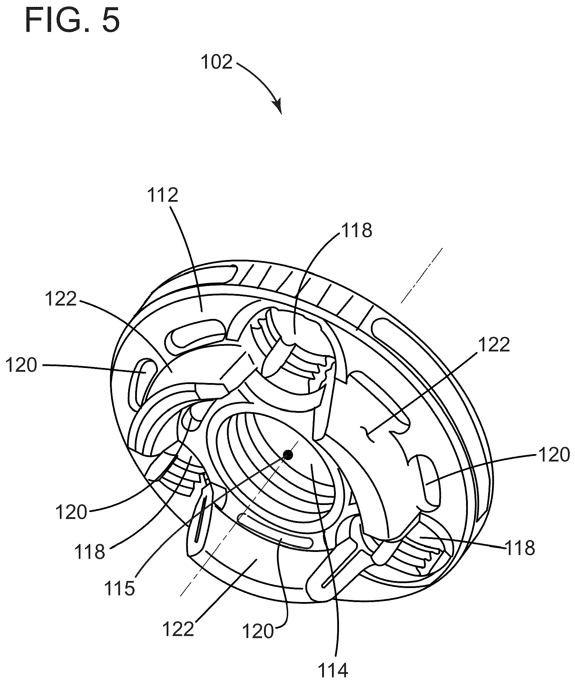

Referring to FIGS. 4 and 5, a top perspective view (FIG. 4) and a bottom perspective view (FIG. 5) of the base plate 102 of implant system 100 is depicted, in accordance with an aspect of the present invention. The base plate 102 may have, for example, a cylindrical shape with a circular top surface 110 and a circular bottom surface 112.

The base plate 102 may include a threaded central bore 114 disposed or positioned in a central region 115 of the base plate 102. The base plate 102 also includes at least one thru-hole. In this particular example, the base plate 102 includes two types of thru-holes, i.e., a plurality of threaded peripheral holes 118 and a plurality of arcuate slots 120. Both the plurality of peripheral holes 118 and the plurality of arcuate slots 120 are disposed circumferentially around the central bore 114 and/or central region 115 of the base plate.

Though only two types of thru-holes are illustrated in the examples herein (i.e., peripheral holes 118 and arcuate slots 120), other types of thru-holes are within the scope of the present invention. For example, the thru-holes may be one or more unthreaded holes or straight slots drilled through the base plate 102.

The plurality of threaded peripheral holes 118 are configured to engage with peripheral screws 154 (see FIGS. 19 and 20). During operation, at least one peripheral screw 154 extends through any one of the plurality of peripheral holes 118 to further anchor the base plate 102 to a bone. As shown in FIG. 20, the peripheral screws 154 include first threads 156 and second threads 158. The first threads 156 are configured to engage with threads of any peripheral hole 118 of the plurality of peripheral holes 118. The second threads 158 are configured to screw into the bone and help anchor the base plate 102 to the bone.

FIG. 5 shows the base plate 102 having a plurality of arcuate protrusions 122 extending from the bottom surface 112 of the base plate 102 that are also disposed circumferentially around the central bore 114. The protrusions 122 may be comprised of a porous metal structure to provide sites for bone in growth when inserted into a prepared glenoid cavity.

As seen in FIG. 5, the plurality of arcuate slots 120 may be positioned adjacent to the inner and outer side walls of the protrusions 122 and my follow the profile of the protrusions 122. The arcuate slots 120, may potentially be utilized for revising the implant system 100, if required. For example, the arcuate slots 120 may allow a surgical instrument, such as an osteotome, to be passed down through the slots 120 to disengage away the metal to bone bond to allow the implant system 100 to be removed during a revision operation.

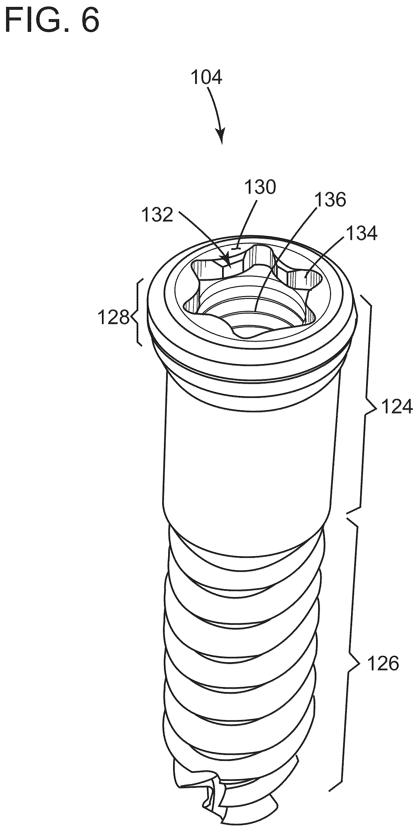

Referring to FIG. 6, a side perspective view of the central screw 104 of the orthopedic implant system 100 is depicted, in accordance with an aspect of the present invention. The central screw 104 is operable to extend through the central bore 114 and threadingly anchor the base plate 102 to a bone (see FIG. 18). In this example, the bone that the central screw 104 engages with is the glenoid cavity of a scapula (see FIG. 18).

As shown in FIG. 6, the central screw 104 may include a proximal non-threaded section 124 and a distal threaded section 126. The proximal section 124 may include a flared head portion 128 positioned at a first end 130 of the central screw 104. The proximal section 124 may also include a recess 132 extending into the proximal section 124 from the first end 130 of the central screw 104.

FIG. 6 shows that the recess 132 may include a first socket portion 134 and a second female threaded portion 136. The first socket portion 134 may extend from the first end 130 of the central screw 104 toward the second female threaded portion 136. The second female threaded portion 136 may extend from a bottom end of the first socket portion 134 toward the distal threaded section 126. The first socket portion 134 may be, for example, a driver feature for engaging a tool for inserting or removing the central screw 104. The second female threaded portion 136 may be, for example, threaded for receiving corresponding threads of a post 152 (see FIG. 19). The proximal section 124 may have, for example, a texture or coating to provide for porous fixation. The proximal section 124 may be, for example, configured or sized and shaped to conserve bone. The distal threaded section 126 may be, for example, threaded to engage a patient's bone to secure the base plate 102 to the patient's bone, such as, the glenoid cavity 12.

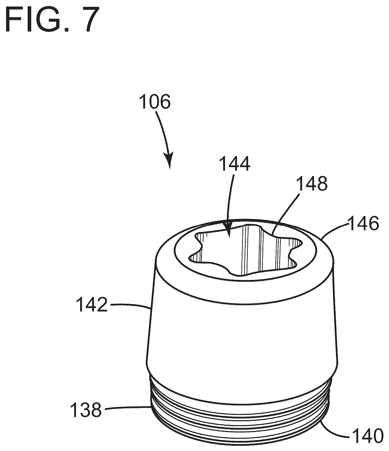

Referring to FIG. 7, a side perspective view of coupling member 106 of the orthopedic implant system 100 is depicted, in accordance with an aspect of the present invention. The coupling member 106 engages the base plate 102 via a threaded portion 138 to secure the central screw 104 within the central bore 114 of the base plate 102.

FIG. 7 shows the coupling member 106 including a distal male threaded section 138 terminating at a bottom end 140 of the coupling member 106. The coupling member 106 may also include a proximal unthreaded section 142 having a central thru-hole 144 extending through the coupling member 106 from a top end 146 to the bottom end 140. The central thru-hole 144 may include a socket portion 148 extending through at least a portion of the central thru-hole 144 from the top end 148. The socket portion 148 may be, for example, a drive feature for engaging a tool for inserting or removing the coupling member 106 into the central bore 114 of the base plate 102. The male threaded section 138 may be, for example, threaded for receiving corresponding female threads within the central bore 114. When threaded into the central bore 114, the bottom end 140 of coupling member 106 may abut against the flared head portion 128 of the central screw 104 to secure and lock the central screw 104 into place.

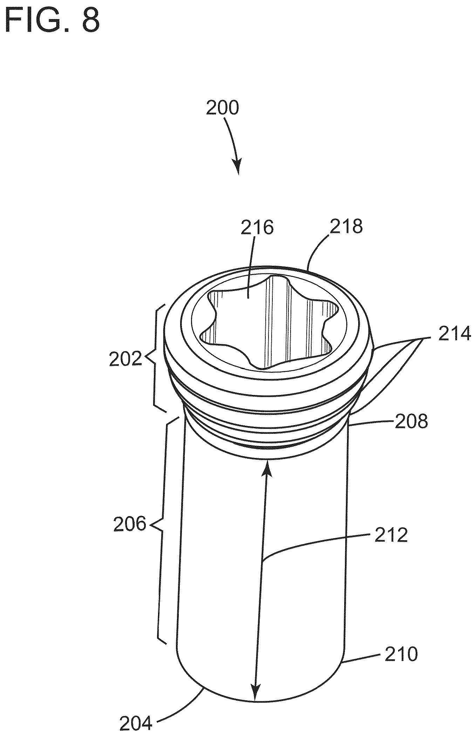

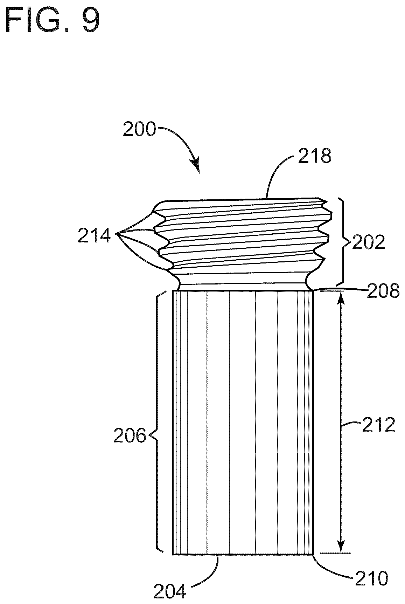

Referring to FIGS. 8 and 9, a top perspective view (FIG. 8) and a side view (FIG. 9) of a first example of an augmentation device 200 of the orthopedic implant system 100 is depicted in accordance with an aspect of the present invention. The augmentation device 200 includes a fastener portion 202 and a footing portion 204 connected therebetween by a post 206.

The augmentation device 200 is operable to extend through at least one thru-hole 118 of the base plate 102. In the examples illustrated herein, the augmentation device 200 is configured to extend through any one of the peripheral holes 118. However, the augmentation device 200 may be configured to extend through any one of the arcuate slots 120. Moreover, the augmentation device 200 may be configured to extend through any other type of thru-hole disposed within the base plate 102.

As shown in FIG. 8, the post 206 includes a first end 208, a second end 210 and a length 212 sized to enable the augmentation device 200 to traverse or fill a defect in a bone. For example, the length 212 of post 206 may be long enough such that when the augmentation device 200 is fastened to base plate 102 and anchored into the scapula 10, the augmentation device 200 may extend into the defect 14 of glenoid cavity 12 to abut its footing portion 204 against a bottom surface of the bone within the defect 14 (see FIG. 18) and provide support to the base plate 102. The post 206 may include a porous surface to provide sites for bone ingrowth when inserted into a prepared glenoid cavity. The porous surface may be comprised of, for example, a porous metal structure, or a porous coating. Though the post 206 is illustrated in FIGS. 8 and 9 as being cylindrical in shape, the post 206 may be configured in other shapes as well. For example, and without limitation, the post 206 may have a square or rectangular cross-sectional shape. Alternatively, the post 206 may have a shape that conforms to the shape of the thru-hole it is extended though.

FIG. 8 also shows the fastener portion 202 as being positioned on the first end 208 of the post 206. The fastener portion 202 couples the augmentation device 200 to the base plate 102. For example, the fastener portion 202 may include male threads 214 that are configured to engage with the female threads on any one of the peripheral holes 118 of the base plate 102. However, the fastener portion may also be configured to engage with any one of the arcuate slots 120 of the plurality of arcuate slots 120. For example, a first arcuate slot 120 may have portions of female threads 214 disposed on opposing side walls of the first arcuate slot 120 for engagement with male threads disposed on the fastener portion 202. Moreover, the fastener portion 202 may be configured to engage with any other thru-hole found in the base plate 102. Additionally, the fastener portion 202 may not include threads, and may be configured to be press-fit or cemented into a thru-hole.

The fastener portion 202 may include various components that are resilient, or spring loaded, and are capable of snapping into place and securing the augmentation device 200 when it is in a predetermined position. For example, the fastener portion 202 may include a top rim disposed at the top end 218 of the fastener portion 202 and a well-known spring loaded retractable retaining ring (or snap ring) disposed in a groove on the lower end of fastener portion 202 adjacent the top first end 208 of the post 206. The retaining ring (not shown) may retract radially into the groove when it is being inserted through a top of a thru-hole, and radially snap back partially out of the groove when it exits the bottom of the thru-hole. The retaining ring and top rim may be spaced apart to capture the base plate between them and to hold the augmentation device 200 in place once the augmentation device 200 extends through the thru-hole.

A socket portion 216 may be recessed into a top end 218 of the fastener portion 202. The socket portion 216 may be, for example, a drive feature for engaging a tool for inserting or removing the augmentation device 200. In this particular example, a tool may engage the socket portion 216 to thread the male threads 214 of the fastener portion 202 into the female threads on any one of the peripheral holes 118 of the base plate 102.

The footing portion 204 is positioned on the second end 210 of the post 206. The footing portion 204 is operable to abut against a surface of the bone within the defect 14 when the fastener portion 202 is fastened to the base plate 102. In the example illustrated in FIGS. 8 and 9, the footing portion 204 comprises a flat bottom footing portion. That is, the footing portion 204 is the flat surface at the bottom second end 210 of the post 206. The flat bottom footing portion 204 is operable to pressingly engage and not penetrate the surface of the bone within the defect 14.

As will be described in greater detail herein, a flat surface 344 may be reamed into a portion of the surface of the bone within the defect 14, directly under the thru-hole that the augmentation device 200 is inserted into. The flat surface 344 may rest against the flat bottom footing portion 204 of augmentation device 200 to provide support for the footing portion 204 along substantially the entire surface area of the flat bottom footing portion 204 and base plate 102.

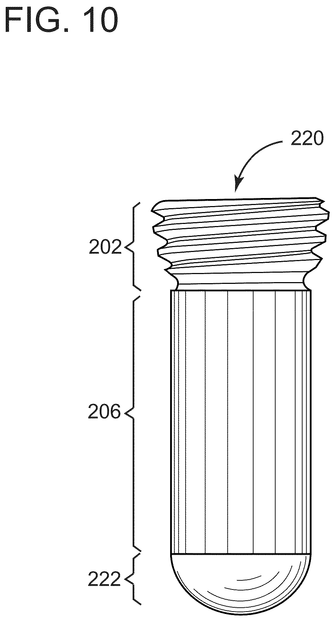

FIG. 10 shows a side perspective view of another example of an augmentation device 220 having an arcuate bottom footing portion 222, in accordance with an aspect of the present invention. The fastener portion 202 and the post 206 of the augmentation device 220 are as described above with reference to the augmentation device 200 and will not be described again here for brevity's sake.

The footing portion 222 has an arcuate or curved shape. The footing portion 222 is configured to press against and not penetrate the surface of the bone within the defect 14. The arcuate bottom footing portion 222 may abut against the bone within the defect 14 over a smaller percentage of its surface area than that of the flat bottom footing portion 204. As such, there may be less reaming required and decreased bone removal, relative to the flat bottom footing portion 204, to prepare the surface of the bone within the defect 14 to receive and support the arcuate bottom footing portion 222.

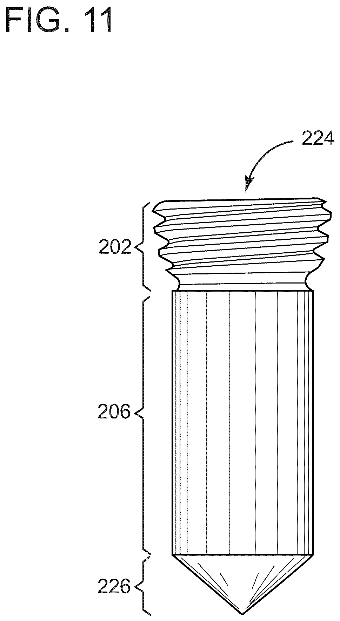

FIG. 11 is a side perspective view of another example of an augmentation device 224 having a conical bottom footing portion 226, in accordance with an aspect of the present invention. The fastener portion 202 and the post 206 of the augmentation device 224 are as described above with reference to the augmentation device 200 and will not be described again here for brevity's sake. The footing portion 226 has a conical shape and again presses against the surface of the bone within the defect 14. The conical bottom footing portion 226 may abut against the bone within the defect 14 over an even smaller percentage of its surface area than that of the flat bottom footing portion 204. As such, there may be less reaming and bone removal required, relative to the flat bottom footing portion 204, to prepare the surface of the bone within the defect 14.

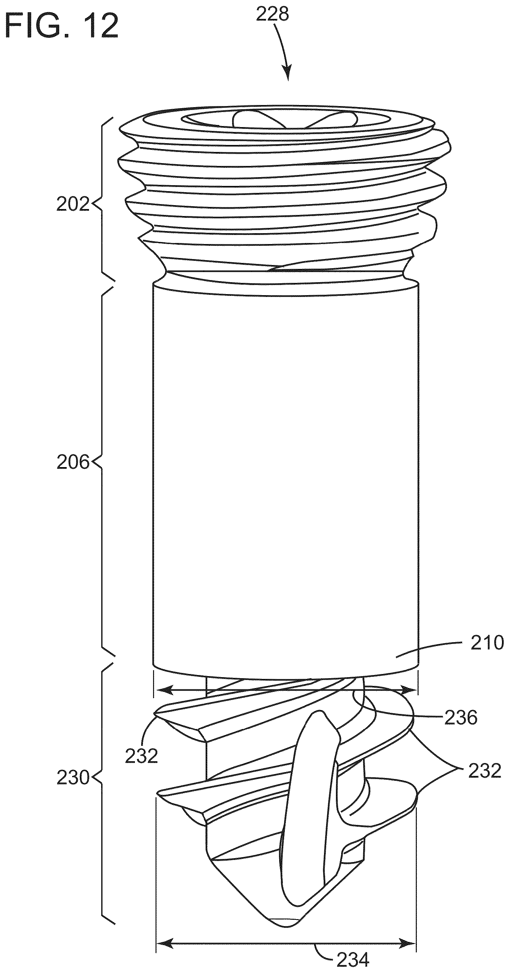

FIG. 12 shows a side perspective view of another example of an augmentation device 228 having a threaded bottom footing portion 230, in accordance with an aspect of the present invention. The fastener portion 202 and the post 206 of the augmentation device 228 are as described above with reference to the augmentation device 200 and will not be described again here for brevity's sake. The threaded bottom footing portion 230 of the augmentation device 228 includes threads 232 that are designed to minimally penetrate the bone within the defect 14. For example, the augmentation device 228 may include at least one thread 232 and have a major diameter 234 that is less than or equal to the outer diameter 236 of the second end 210 of the post 206. Additionally, the threaded bottom footing portion 230 may include a limited number of threads, for example, no more than three threads 232, to limit the penetration into the surface of the bone within the defect 14. The threaded bottom footing portion 230 is operable to be threaded into the surface of the bone within the defect 14.

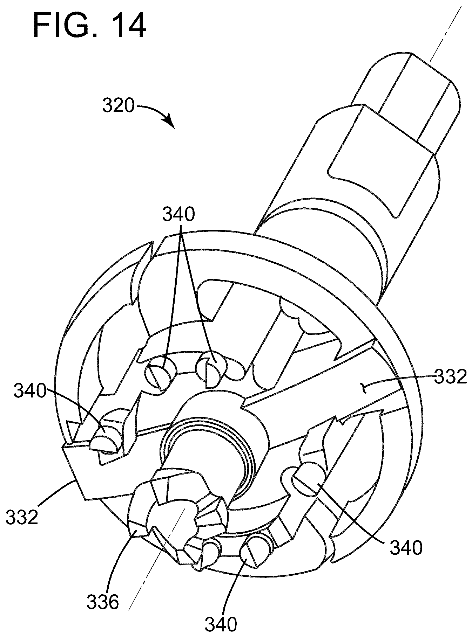

Referring to FIGS. 13-18, various steps utilized in a method of preparing a bone for the orthopedic implant system 100 are depicted in accordance with aspects of the present invention. In this particular case, the method will be used to implant the orthopedic implant system 100 in the glenoid cavity 12 of a scapula 10. However, the method may be used to implant other orthopedic implant systems within the scope of this invention into other bones as well. More specifically, FIG. 13 depicts a block diagram of a method of surgically preparing a bone to receive the orthopedic implant system in accordance with the present invention and FIGS 14-21 support the various steps of the method.

Referring to FIG. 14, a bottom perspective view of a bone reamer 320 used to prepare a top (or outer) surface 322 of a glenoid cavity 324 having a bone defect 328, such as a void, to accept the orthopedic implant system 100 is depicted, in accordance with an aspect of the present invention. Similar such bone reamers are disclosed in: international application no. PCT/US2019/051322, filed on September 16, 2019, which is incorporated herein by reference in its entirety.

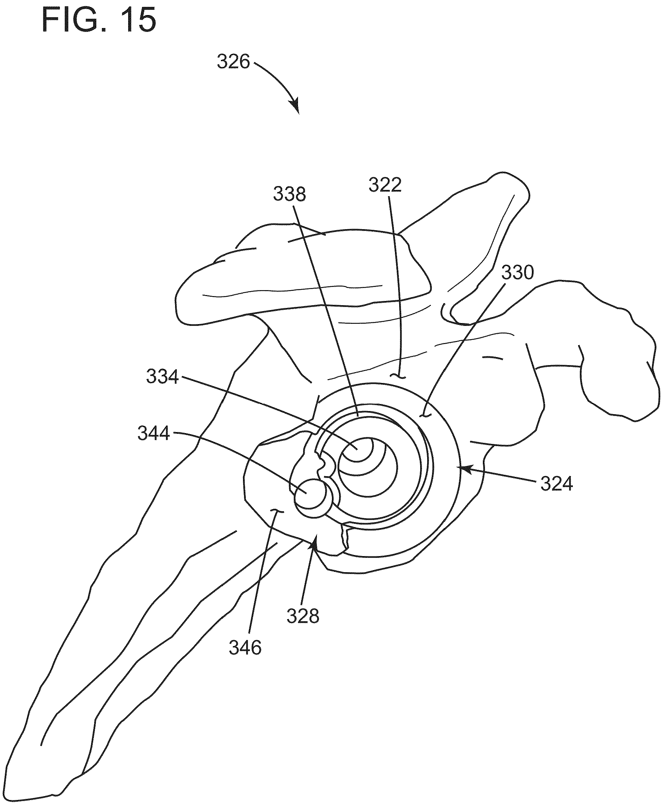

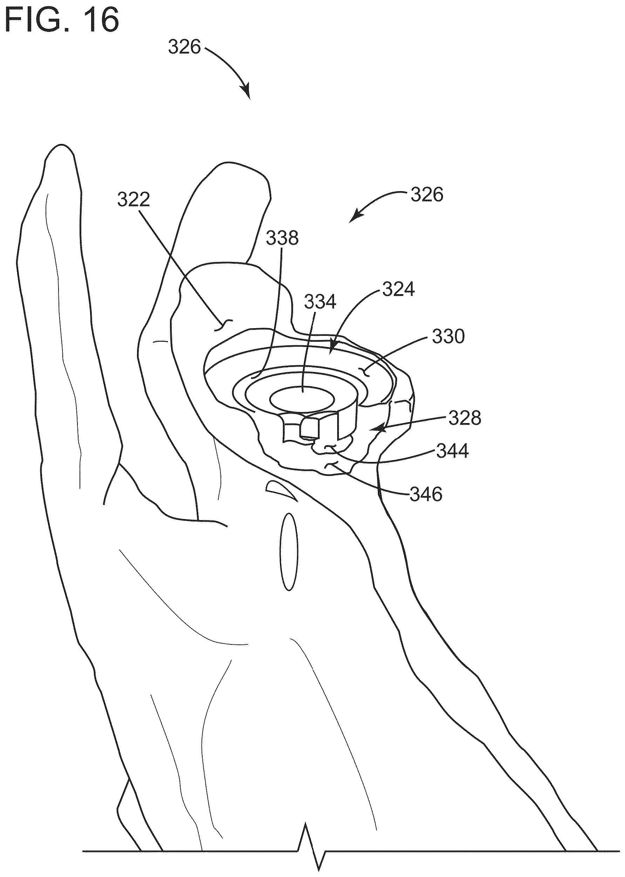

Referring also to FIGS. 15 and 16, a top perspective view (FIG. 15) and side perspective view (FIG. 16) of the defective glenoid cavity 324 is depicted, in accordance with an aspect of the present invention. In FIGS. 15 and 16, the outer surface 322 of the glenoid cavity 324 has been prepared to accept an orthopedic implant system 100 with bone reamer 320.

After surgically exposing the outer surface 322 of the glenoid cavity 324 of a patient (ref. 300 in FIG. 13), the bone reamer 320 may be used to remove a portion of a circular surface 330 into the outer surface 322 of the glenoid cavity 324 (ref. 302 in FIG. 13). The reaming of the portion of the circular surface 330 may be done with the blade member 332 of the bone reamer 320. The portion of the circular surface 330 may, or may not, be a complete circular surface due to bone loss caused by the bone defect 328.

Additionally, the bone reamer 320 may be used to drill a center bore hole 334 into the glenoid cavity 324 that is concentric with the portion of the circular surface 330 (ref. 304 in FIG. 13). The drilling of the center bore hole 334 may be done with the center drill bit 336 of the bone reamer 320.

The bone reamer 320 may cut a portion of a circular groove 338 into the glenoid cavity bone 324 that is concentric with the center bore hole 334 (ref. 306 in FIG. 13). The cutting of the portion of the circular groove 338 may be done with the plurality of cutting pegs 340 of the bone reamer 320. The portion of the circular groove 338 may, or may not, be a complete circular groove for a full 360 degrees due to pre-existing bone loss.

The bone reamer 320 is capable of reaming the portion of the circular surface 330, drilling the center bore hole 334 and cutting the portion of the circular groove 338 substantially simultaneously. However, the portion of the circular surface 330, the center bore hole 334 and the portion of the circular groove 338 may be machined into the glenoid cavity 324 in any sequence using any number of instruments.

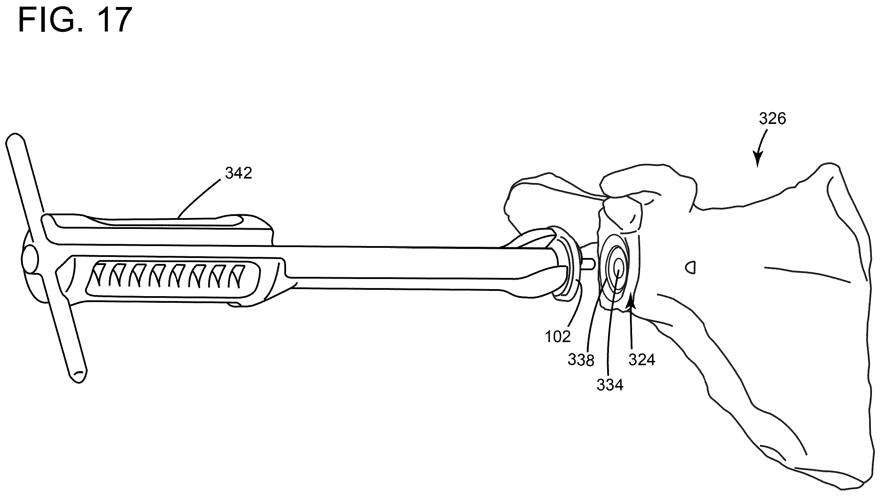

FIG. 17 shows a side perspective view of the base plate 102 of orthopedic implant system 100 being inserted into the prepared glenoid cavity 324 with an insertion tool 342 is depicted, in accordance with an aspect of the present invention. The insertion tool 342 may be used to orient a thru-hole of the base plate 102 of the implant system 100 over the defect 328 in the glenoid cavity bone 324 (ref. 308 in FIG. 13).

Thereafter, the insertion tool 342 may be used to insert the base plate 102 into the center bore hole 334 and the portion of the circular groove 338, such that the thru-hole of the base plate 102 is positioned directly over the defect 324 (ref. 310 in FIG. 13). More specifically, the central bore 114 of the base plate 102 may be positioned, by the insertion tool, directly over the center bore hole 334 within the glenoid cavity 324 and the central screw 104 may be used to anchor the base plate 102 to the glenoid cavity 324 of scapula 326. The arcuate protrusions 122 of the base plate 102 may be press-fit into the circular groove 338 to further anchor the base plate 102 to the glenoid cavity 324. One or more peripheral screws 154 (see FIGS. 19 and 20) may then be used to further anchor the base plate 102 to the glenoid cavity 324.

Thereafter, a flat surface 344 may be reamed into a portion of the surface 346 of the glenoid cavity bone 324 within the defect 328 directly under the thru-hole (ref. 312 in FIG. 13). The reaming may be done by any one of several well-known reaming tools.

Alternatively, no reaming of a surface 344 may be required. This may particularly be the case wherein the augmentation device 200 has an arcuate bottom footing portion 222, a conical bottom footing portion 226 or a threaded bottom footing portion 230.

FIG. 18 shows the orthopedic implant system 100 being implanted into the glenoid cavity 324, in accordance with an aspect of the present invention. Thereafter, the augmentation device 200 extends through the thru-hole (ref. 314 in FIG. 13).

The augmentation device 200 may be fastened to the base plate 102 such that the footing portion 204 of the augmentation device 200 abuts against a surface 346 of the defect 328, and does not penetrate the surface 346 of the glenoid cavity bone 324 (ref. 316 in FIG. 13). More specifically in this case, the augmentation device 200 may be fastened to the base plate 102 by coupling the fastener portion 202 to the peripheral thru-hole 118 such that the foot portion 204 of the augmentation device 200 abuts against the flat surface 344 (see FIGS. 15 and 16) reamed into the surface 346 of the glenoid cavity bone 324 within the defect 328.

When implanted, the augmentation device 200 provides support to the base plate 102 and allows for attachment to the glenoid cavity 324. The augmentation device 200 requires minimal removal of the surface bone within the defect 328. Additionally, the augmentation device 200 allows the same base plate 102 to be used regardless of the deficiencies in the bone. Once the base plate 102 is implanted, then one or more augmentation devices 200 may be inserted depending on the type and severity of the defects encountered.

Referring to FIGS. 19 and 20, a top perspective view (FIG. 19) and a bottom perspective view (FIG. 20) of the orthopedic implant system 100 is depicted in accordance with an aspect of the present invention. Thereafter, a glenosphere 150 may be disposed over the base plate 102 of the implant system 100 for use in a reverse shoulder arthroplasty (ref. 318 in FIG. 13).

The glenosphere 150 may be connected to the base plate 102 by a threaded post 152, which may extend through the coupling member 106 (see FIG. 7) and thread into the female threaded portion 136 of the recess 132 of the central screw 104 (see FIG. 6). The central screw 104 and peripheral screws 154 may be used to anchor the implant system 100 into the scapula 326. The peripheral screws 154 may include first threads 156 configured to engage with threads of the peripheral holes 118 and second threads 158 configured to screw into the scapula 326. The augmentation device 200 may provide support for the base plate 102 when implanted into the scapula 326.

Referring to FIG. 21, a top perspective view of the orthopedic implant system 100 is depicted in accordance with an aspect of the present invention. A glenoid liner 160 may be connected to the base plate 100 rather than the glenoid sphere 150 (ref. 318 in FIG. 13). The glenoid liner 160 may be for use in an anatomic shoulder arthroplasty.

The glenoid liner 160 may be connected to the base plate 102 using several different attaching techniques (not shown). For example, the glenoid liner 160 may have protrusion that match the arcuate slots 120 of the base plate 102, such that the protrusions may be press fit into the slots 120. Alternatively, the glenoid liner 160 may have a flanged end that extends radially inward. The flanged end may cup under the lower surface 112 of the base plate 102 to secure the liner 160 to the base plate 102. To accommodate the glenoid liner 160, the coupling member 106 may be reduced in height so that its top surface is flush, or nearly flush, with the top surface 110 of the base plate, once the coupling member 106 is attached to the base plate 102.

Referring to FIGS. 22 and 23, an example is depicted of a side perspective view (FIG. 22) and a side exploded view (FIG 23) of another example of an orthopedic implant system 400 in accordance with the present invention. In contrast to the implant system 100, the base plate 402, central screw 404 and coupling member 406 are integrally connected as a single monobloc construct. In this implant system 400, there is no central bore 114 (see FIG. 4) that is positioned in the central region 115 of the base plate 102 and to which both the central screw 104 and coupling member 106 may be removably connected. Rather, in implant system 400, the central screw 404 extends from a lower surface 410 of the central region 415 of the base plate 402 and the coupling member 406 extends from a top surface 408 of the central region 415 of the base plate 402. The base plate 402, central screw 404 and coupling member 406 may be cast and/or machined from a single block of material. All other aspects of the implant system 100 and the implant system 400 (including the augmentation devices, such as augmentation device 200) are substantially similar.

As may be recognized by those of ordinary skill in the art based on the teachings herein, numerous changes and modifications may be made to the above-described and other embodiments of the present disclosure without departing from the scope of the disclosure. The components of the implant systems, devices, and/or systems as disclosed in the specification, including the accompanying abstract and drawings, may be replaced by alternative component(s) or feature(s), such as those disclosed in another embodiment, which serve the same, equivalent or similar purpose as known by those skilled in the art to achieve the same, equivalent or similar results by such alternative component(s) or feature(s) to provide a similar function for the intended purpose. In addition, the implant systems, devices, and/or systems may include more or fewer components or features than the embodiments as described and illustrated herein. For example, the components and features of implant system 100 may be used interchangeably and in alternative combinations as would be modified or altered by one of skill in the art. Further, the steps of the surgical methods associated with the implant system 100 may be used interchangeably and in alternative combinations as would be modified or altered by one of skill in the art. Accordingly, this detailed description of the currently-preferred embodiments is to be taken in an illustrative, as opposed to limiting of the disclosure.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the operational embodiments described herein are exemplary of a plurality of possible arrangements to provide the same general features, characteristics, and general system operation. Modifications and alterations will occur to others upon a reading and understanding of the preceding detailed description. It is intended that the invention be construed as including all such modifications and alterations.

### Numbered Examples

Having thus described the preferred embodiments, the invention is now described by way of non-limiting numbered examples:
1. An implant system comprising:
   a base plate having at least one thru-hole;
   a central screw operable to extend from a lower surface of the base plate and threadingly anchor the base plate to a bone; and
   at least one augmentation device extending through the at least one thru-hole, the augmentation device comprising:
      a post having a first end, a second end and a length sized to enable the augmentation device to extend into a defect in the bone when the base plate is anchored to the bone,
      a fastener portion positioned on the first end of the post, the fastener portion couples the at least one augmentation device to the base plate, and
      a footing portion positioned on the second end of the post, the footing portion being configured to press against a bone surface within the defect when the fastener portion is coupled to the base plate.
2. The implant system of example 1, comprising:
   a central bore disposed in the base plate;
   wherein the central screw couples and extends through the central bore.
3. The implant system of example 1, wherein the base plate and central screw are integrally connected as a single monobloc construct.
4. The implant system of example 1, wherein the footing portion of the augmentation device comprises any one of a flat bottom, an arcuate bottom and a conical bottom, the footing portion is operable to press against the surface of the bone within the defect.
5. The implant system of example 1, wherein the footing portion of the augmentation device comprises a threaded bottom footing portion, the threaded bottom footing portion having at least one thread, wherein the at least one thread has a major diameter that is smaller than an outer diameter of the second end of the post, the threaded bottom footing portion being configured to thread into the bone surface within the defect.
6. The implant system of example 1, comprising:
   the at least one thru-hole comprising a plurality of threaded peripheral holes disposed circumferentially around the central bore; and
   the fastener portion comprising a threaded fastener portion having threads configured to engage with the threads of a first peripheral hole of the plurality of peripheral holes.
7. The implant system of example 6, comprising:
   at least one peripheral screw operable to extend through any one of the plurality of peripheral holes, the at least one peripheral screw comprising:
   first threads configured to engage with threads of a second peripheral hole of the plurality of peripheral holes, and
   second threads configured to screw into the bone.
8. The implant system of example 1, comprising:
   the at least one thru-hole comprising a plurality of arcuate slots disposed circumferentially around the central bore; and
   the fastener portion configured to engage with a first arcuate slot of the plurality of arcuate slots.
9. The implant system of example 8, comprising:
   the first arcuate slot having portions of female threads disposed on opposing side walls of the first arcuate slot; and
   the fastener portion comprising male threads configured to engage with the portions of female threads of the first arcuate slot.
10. The implant system of example 1, comprising one of a glenosphere and a glenoid liner operable to be attached to the base plate after the base plate is anchored to the bone.
11. An implant system comprising:
   a base plate having a plurality of threaded peripheral holes disposed circumferentially around a central region of the base plate;
   a central screw operable to extent from a lower surface of the base plate and threadingly anchor the base plate to a bone;
   at least one augmentation device operable to extend through a first peripheral hole of the plurality of threaded peripheral holes, the augmentation device comprising:
      a post having a first end, a second end and a length sized to enable the augmentation device to extend into a defect in the bone when the base plate is anchored to the bone,
      a threaded fastener portion positioned on the first end of the post, the fastener portion having threads configured to engage with the threads of the first peripheral hole; and
      a footing portion positioned on the second end of the post, the footing portion operable to abut against the bone within the defect.
12. The implant system of example 11, comprising:
   a central bore disposed in the base plate;
   wherein the central screw is separate from the base plate and operable to extend through the central bore.
13. The implant system of example 11, wherein the base plate and central screw are integrally connected as a single monobloc construct.
14. The implant system of example 11, wherein the footing portion of the augmentation device comprises at least one flat bottom, an arcuate bottom and a conical bottom, the footing portion configured to pressingly engage the bone within the defect.
15. The implant system of example 11, wherein the footing portion of the augmentation device comprises a threaded bottom footing portion, the threaded bottom footing portion having at least one thread, the at least one thread having a major diameter that is smaller than an outer diameter of the second post, the threaded bottom footing portion operable to thread into the surface of the bone within the defect.
16. The implant system of example 11, comprising:
   at least one peripheral screw operable to extend through any one of the plurality of peripheral holes, the at least one peripheral screw comprising:
   first threads configured to engage with threads of a second peripheral hole of the plurality of peripheral holes, and
   second threads configured to screw into the bone.
17. The implant system of example 11 comprising at least one of a glenosphere and a glenoid liner, wherein the glenosphere and glenoid liner are coupled to the base plate after the base plate is anchored to the bone.
18. An augmentation device of an implant system, the implant system having a base plate that is operable to be anchored to a bone, the augmentation device comprising:
   a post configured to extend through a thru-hole of the base plate, the post having a first end and a second end, wherein the augmentation device is sized to extend into a defect in the bone when the base plate is anchored to the bone,
   a fastener portion positioned at the first end of the post, the fastener portion operable to couple to the base plate, and
   a footing portion positioned at the second end of the post, the footing portion configured to press against a surface of the bone within the defect when the fastener portion is coupled to the base plate.
19. The augmentation device of example 18, wherein the footing portion of the augmentation device comprises at least one of a flat bottom, an arcuate bottom and a conical bottom, the footing portion being configured to contact the bone surface within the defect
20. The augmentation device of example 18, wherein the footing portion of the augmentation device comprises a threaded bottom footing portion, the threaded bottom footing portion having at least one thread, the at least one thread having a major diameter that is smaller than an outer diameter of the second end of the post, the threaded bottom footing portion operable to thread into the bone surface within the defect.
21. The augmentation device of example 18, wherein the fastener portion comprises a threaded fastener portion having threads configured to engage with threads of a peripheral hole of the base plate.
22. The augmentation device of example 18, wherein the fastener portion is configured to engage with an arcuate slot of the base plate.
23. A method of surgically implanting an implant system into a bone, the method comprising:
   surgically exposing an outer surface of a bone, the outer surface having a bone defect;
   reaming a portion of a circular surface into the outer surface of the bone;
   drilling a bore into the bone that is concentric with the portion of the circular surface;
   orienting a thru-hole of a base plate of an implant system over the defect in the bone;
   inserting the base plate into the bore hole, such that the thru-hole of the base plate aligns over the defect;
   extending an augmentation device through the thru-hole;
   fastening the augmentation device to the base plate such that a footing portion of the augmentation device abuts against a surface of the bone within the defect; and
   attaching at least one of the glenosphere and glenoid liner to the base plate.
24. The method of example 23, comprising:
   cutting a portion of a circular groove into the bone that is concentric with the bore hole; and
   inserting a portion of the base plate into the portion of the circular groove.
25. The method of example 23, comprising:
   reaming a surface into a portion of the bone surface within the defect under the thru-hole; and
   fastening the augmentation device to the base plate such that the foot portion of the augmentation device abuts the surface.
26. The method of the example 23, wherein the portion of the bone is a glenoid cavity of a scapula.
27. The method of example 23, wherein the thru-hole is a threaded hole of the base plate.

## Claims

1. An implant system comprising:
a base plate having at least one thru-hole;
a central screw extending from a lower surface of the base plate and operable to threadingly anchor the base plate to a bone; and
at least one augmentation device extending through the at least one thru-hole, the augmentation device comprising: (i) a post having a first end, a second end and a length sized to enable the augmentation device to extend into a defect in the bone when the base plate is anchored to the bone, (ii) a fastener portion positioned on the first end of the post, the fastener portion is secured to the base plate, and (iii) a footing portion positioned on the second end of the post, the footing portion comprising a flat bottom configured to press against a bone surface within the defect when the fastener portion is coupled to the base plate.

2. The implant system of claim 1, comprising:
a central bore disposed in the base plate;
wherein the central screw couples and extends through the central bore.

3. The implant system of claim 1 or 2, wherein the base plate and central screw are integrally connected as a single monolithic construct.

4. The implant system of claim 1, 2 or 3, wherein the footing portion of the augmentation device comprises a threaded bottom footing portion, the threaded bottom footing portion having at least one thread, wherein the at least one thread has a major diameter that is smaller than an outer diameter of the second end of the post, the threaded bottom footing portion being configured to thread into the bone surface within the defect.

5. The implant system of any one of claims 1 to 4, comprising:
the at least one thru-hole comprising a plurality of threaded peripheral holes disposed circumferentially around the central bore; and
the fastener portion comprising a threaded fastener portion having threads configured to engage with the threads of a first peripheral hole of the plurality of peripheral holes.

6. The implant system of claim 5, comprising:
at least one peripheral screw operable to extend through any one of the plurality of peripheral holes, the at least one peripheral screw comprising:
first threads configured to engage with threads of a second peripheral hole of the plurality of peripheral holes, and
second threads configured to screw into the bone.

7. The implant system of any preceding claim, comprising:
the at least one thru-hole comprising a plurality of arcuate slots disposed circumferentially around the central bore; and
the fastener portion configured to engage with a first arcuate slot of the plurality of arcuate slots.

8. The implant system of claim 7, comprising:
the first arcuate slot having portions of female threads disposed on opposing side walls of the first arcuate slot; and
the fastener portion comprising male threads configured to engage with the portions of female threads of the first arcuate slot.

9. The implant system of any preceding claim comprising one of a glenosphere and a glenoid liner operable to be attached to the base plate after the base plate is anchored to the bone.

10. An implant system comprising:
a base plate having a plurality of threaded peripheral holes disposed circumferentially around a central region of the base plate;
a central screw extending from a lower surface of the base plate and operable to threadingly anchor the base plate to a bone;
at least one augmentation device operable to extend through a first peripheral hole of the plurality of threaded peripheral holes, the augmentation device comprising: (i) a post having a first end, a second end and a length sized to enable the augmentation device to extend into a defect in the bone when the base plate is anchored to the bone, (ii) a threaded fastener portion positioned on the first end of the post, the fastener portion having threads engaged with the threads of the first peripheral hole; and (iii) a footing portion positioned on the second end of the post, the footing portion comprising a flat bottom operable to abut against the bone within the defect.

11. The implant system of claim 10, comprising:
a central bore disposed in the base plate;
wherein the central screw is separable from the base plate and operable to extend through the central bore.

12. The implant system of claim 10 or **11,** wherein the base plate and central screw are integrally connected as a single monobloc construct.

13. The implant system of claim 10, **11** or 12, wherein the footing portion of the augmentation device comprises a threaded bottom footing portion, the threaded bottom footing portion having at least one thread, the at least one thread having a major diameter that is smaller than an outer diameter of the second end of the post, the threaded bottom footing portion operable to thread into the surface of the bone within the defect.
